Europäisches Patentamt

European Patent Office

Office européen des brevets

(19)

(11) Publication number: **0 019 930**
**B1**

(12) **EUROPEAN PATENT SPECIFICATION**

(45) Date of publication of patent specification: **16.05.84**

(51) Int. Cl.³: **A 61 B 17/38**

(21) Application number: **80103050.3**

(22) Date of filing: **31.05.80**

(54) **High frequency electrosurgical instrument for use in an endoscope.**

(30) Priority: **04.06.79 JP 75744/79**
**13.06.79 JP 80440/79**
**14.06.79 JP 81440/79**

(43) Date of publication of application:
**10.12.80 Bulletin 80/25**

(45) Publication of the grant of the patent:
**16.05.84 Bulletin 84/20**

(84) Designated Contracting States:
**BE CH DE FR GB IT LI NL SE**

(56) References cited:
**DE-A-2 429 462**
**DE-A-2 657 256**
**FR-A- 440 731**
**GB-A- 618 528**

(73) Proprietor: **OLYMPUS OPTICAL CO., LTD.**
**43-2, 2-chome, Hatagaya Shibuya-ku**
**Tokyo 151 (JP)**

(72) Inventor: **Komiya, Osamu**
**23-3, Ohya-machi Hachioji-shi**
**Tokyo (JP)**

(74) Representative: **Freischem, Werner, Dipl.-Ing.**
**et al**
**Patentanwälte Dipl.-Ing. W. Freischem Dipl.-Ing.**
**I. Freischem An Gross St. Martin 2**
**D-5000 Köln 1 (DE)**

Courier Press, Leamington Spa, England.

## Description

This invention relates to a high frequency electrosurgical instrument for use in an endoscope comprising a flexible tube having a distal end portion which has first and second wire guiding holes set apart axially of the tube, the first hole being closer to the distal end than the second hole; and electrode wire extending through said tube, one end of which is fixed within the distal end portion and the other portion of which comes out of the tube through the first wire guiding hole to extend outside the tube and enters the tube through the second wire guiding hole to extend through the tube toward the proximal end of the tube.

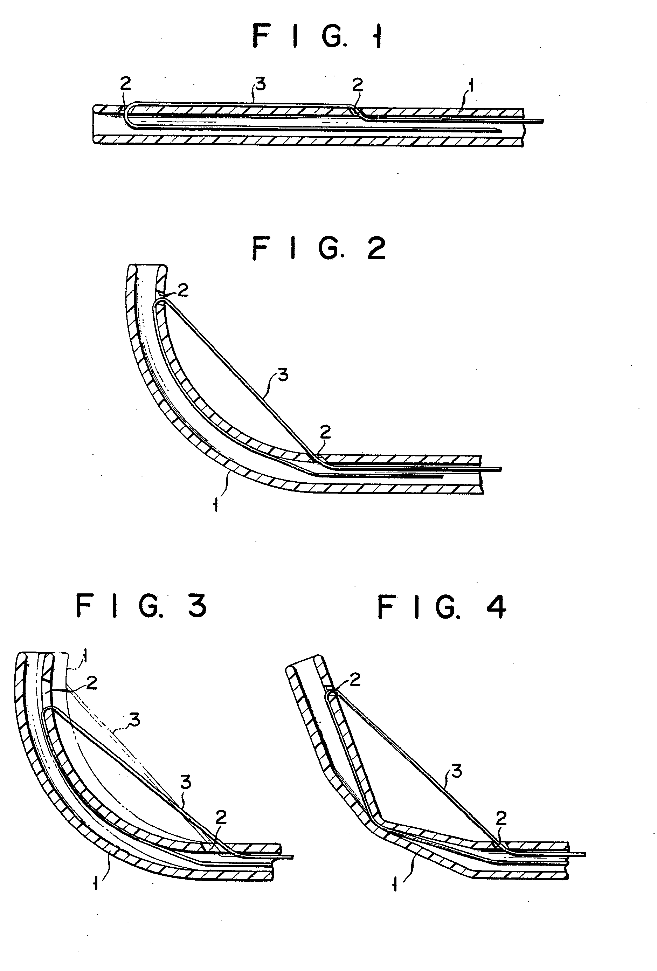

An electrosurgical instrument of this type is known from DE—A—2 657 256 and is shown in Fig. 1. The instrument comprises a flexible tube 1 made of a synthetic resin such as fluorine resin. The tube 1 has at its distal end portion a pair of electrode wire holes 2 which are set apart in the axial direction of the tube 1. An electrode wire 3 extends through the tube 1, comes out of the tube 1 through one of the holes 2 which is remote from the distal end of the tube 1, enters the tube 1 through the other hole 2 and turns back to extend through the tube 1 to the proximal end of the tube 1 to have its end secured to the proximal end portion of the tube 1.

The tube 1 is guided through an endosocpe inserted in a coeliac cavity until its distal end portion is placed near the coeliac cavity. Then, an operation section provided at the proximal end of the tube 1 is operated so as to pull the electrode wire 3. As the wire 3 is pulled, the distal end portion of the tube 1 is bent to form a bow with the wire 3 extended as a string, as illustrated in Fig. 2. Alternatively, the operation section is operated to push the electrode wire 3 such that the distal end portion of the wire 3 is arcuated. The straightened or curved distal end portion of the wire is brought into contact with an affected part in the coeliac cavity. A high frequency current is applied between the electrode wire 3 and the patient so that the affected part is cut.

The known electrosurgical instrument is disadvantageous in some respects. First, if the electrode wire 3 is pulled too much, the distal end portion of the tube 1 is collapsed at the middle point as shown in Fig. 4. Secondly, the distal end of the tube 1 may collapse if a large external force exerts on its outer periphery. Thirdly, when the distal end portion of the tube 1 is bent as shown in Fig. 2 and when a high frequency current is supplied, the heated and tightened electrode wire 3 will likely to cut the tube 1, thus forming slits extending from the holes in the axial direction of the tube 1. If this happens, the wire 3 outside the tube moves from the position shown in Fig. 2 to the position shown in Fig. 3, making it difficult to cut an affected part at the desired portion.

Some problems arise also in case the electrode wire 3 is pushed into the tube 1 so that its portion outside the tube 1 is curved in the form of an arc. First, as it is repeatedly pushed into, and pulled out of, the tube 1 through the holes 2, the electrode wire 3 is likely to cut the tube 1 in the same manner as mentioned above particularly when it is heated. If the slits are formed, it becomes difficult to fully extend the electrode wire 3 from the lateral wall of the distal end portion of the tube 1. Secondly, the portion of the electrode wire 3, if formed in the shape of an arc outside the tube 1, may not project in the desired direction or may move to a position other than the desired position when it touches the inner wall of a coeliac cavity. Much skill is therefore required of the operator in order to cut an affected part existing in a narrow, tubular coeliac cavity.

From F—A—440 731 is known a probe or injecting tube for insertion into body passages. The wall of the tube is made of rubber or caoutchouc. To prevent collapsing of the probe or tube a spiral wire or coil is arranged within the rubber or caoutchouc wall of the tube.

The invention is based on the problem to improve the electrosurgical instrument described in the beginning in that way that the disadvantages described are avoided. The instrument shall thus be constructed in that way that the flexible tube can never be so cut by an electrode wire that there are formed slits extending axially from electrode wire guiding holes, as the electrode wire is repeatedly pushed and pulled and a collapsing of the distal end portion of the tube is prevented.

This problem is solved according to the invention that an elastic helical coil whose cross sectional profile is complementary to that of the interior of said distal end portion is provided in the distal end portion so as to prevent the distal end portion from being permanently deformed and that that portion of said electrode wire which enters the tube passes between first two adjacent turns of the coil, which are separated from each other by a distance larger than the diameter of said electrode wire and then extends through said coil and that said second wire guiding hole extends between said first two adjacent turns of the coil such that the length of the second guiding hole along the tube is larger than the diameter of said electrode wire.

Further features of the invention will be seen from the patent claims.

This invention is described in detail below with reference to the accompanying drawing which illustrate embodiments and in which:

Fig. 1 is a longitudinal cross sectional view of the main part of a high frequency electrosurgical instrument of known type;

Fig. 2 is a longitudinal cross sectional view of the main part of the known instrument, with an electrode wire pulled so that the instrument can be used;

Fig. 3 is a longitudinal cross sectional view of the main part of the known instrument, when the electrode wire cuts a tube, thus forming slits extending respectively from the electrode wire guiding holes of the tube;

Fig. 4 is a longitudinal cross sectional view of the main part of the known instrument, with the tube collapsed at the distal end portion;

Fig. 5 is a longitudinal cross sectional view of an embodiment of a high frequency electrosurgical instrument according to this invention;

Figs. 6 and 7 are longitudinal cross sectional views of the instrument of Fig. 5, illustrating how the main part of the instrument is used, respectively;

Fig. 8 shows how to use the instrument of Fig. 5 in the coeliac cavity;

Figs. 9 to 12 are longitudinal cross sectional views of the main parts of other embodiments of high frequency electrosurgical instruments according to this invention;

Fig. 13 is a longitudinal cross sectional view of another high frequency electrosurgical instrument according to this invention;

Fig. 14 is a cross sectional view taken along line 14—14 in Fig. 13;

Fig. 15 shows the distal end portion of an endoscope and the distal end portion of the instrument of Fig. 13, illustrating how to use the instrument;

Fig. 16 illustrates how to use the instrument of Fig. 13 in a coeliac cavity;

Fig. 17 is a longitudinal cross sectional view of the main part of a modification of the instrument shown in Fig. 13;

Fig. 18 is a longitudinal sectional view of the main part of still another high frequency electrosurgical instrument according to this invention; and

Fig. 19 is a cross sectional view taken along line 24—24 in Fig. 18.

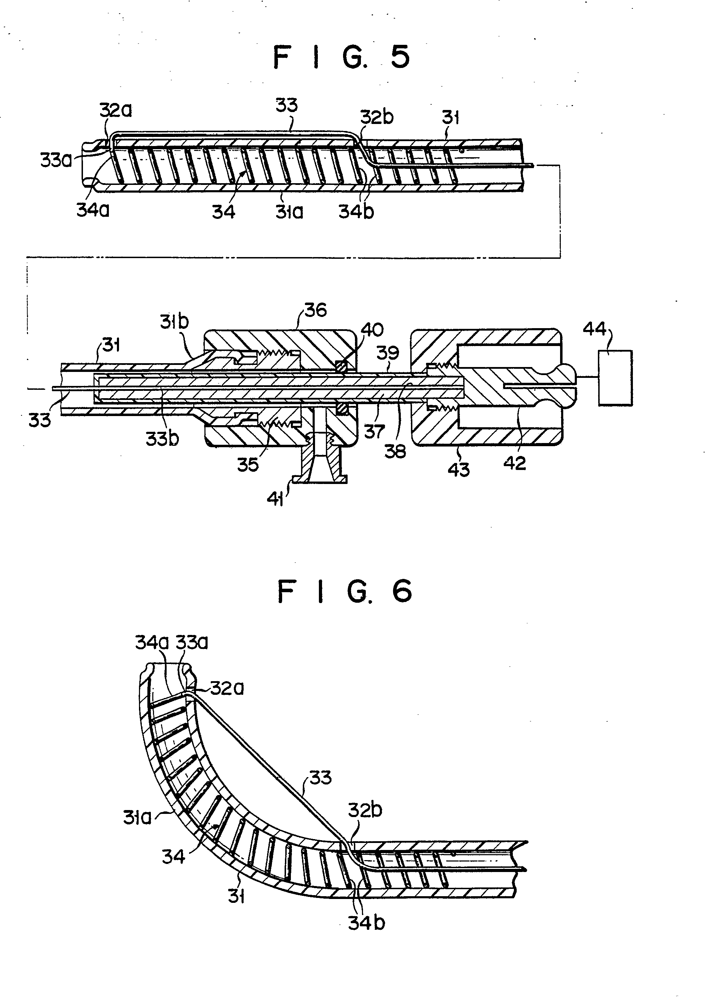

Now referring to Figs. 5 to 8, a high frequency electrosurgical instrument according to this invention will be described. The instrument has a hollow cylindrical tube 31 made of a synthetic resin such as fluorine resin with an outer diameter of 1.5 to 2.5 mm. It is flexible and electrically insulative. The tube 31 has its diameter reduced at the open distal end. Its distal end portion 31a has a pair of holes 32a and 32b for guiding an electrode wire 33. The holes 32a and 32b are set apart in the axial direction of the tube 31.

A flexible reinforcing coil 34 is inserted in the distal end portion 31a of the tube 31. It is disposed coaxially with the tube 31, with its distal end located at the hole 32a and its proximal end located closer to the proximal end of the tube 31 than the hole 32b. The coil 34 is made of an elastic metal wire element (such as a stainless steel wire element) 0.1 to 0.3 mm thick. The pitch of the coil is about three times the diameter of the metal wire. The gap between two adjacent turns of the coil 34 is therefore substantially as twice as the thickness of the wire element.

The distal end 34a of the reinforcing coil 34 is located, as mentioned above, adjacent the hole 32a (hereinafter called "first hole"). The wire 33 is integral with the coil 34 and is thus made of said metal wire element. The wire 33 has one end connected to the distal end 34a of the coil 34. The end of the wire 33 connected to the coil 34 will hereinafter be called the "distal end 33a". The wire 33 comes out of the tube 31 through the first hole 32a, extends outside the tube 31, and enters the tube 31 through the other hole 32b (hereinafter called "second hole"). It further extends through a gap between two adjacent turns 34b of the coil 34 which are located near the second hole 32b. The wire 33 extends through the tube 31 to the proximal end portion 31b of the tube 31.

A tubular connecting member 35 is partly and fixedly inserted in the proximal end portion 31b of the tube 31. The outer periphery of that portion of the connecting member 35 which is outside the tube 31 is put in screw engagement with a hollow cylindrical insulative cover 36. The insulative cover 36 is made of an electrically insulative material such as an electrically insulative synthetic resin. It covers not only the outer periphery of the connecting member 35 but also the outer periphery of the proximal end portion 31b of the tube 31. Through the insulative cover 36 and the connecting member 35 there is provided a metal rod 37 which can be moved in the axial direction of the tube 31. The rod 37 has a central through bore 38 in which the proximal end portion 33b of the electrode wire 33 is held immovably. The wire 33 is thus secured to the metal rod 37. The rod 37 is inserted in an insulative tube 39 made of an electrically insulative material such as synthetic resin and is protected by the insulative tube 39. Further, an O ring 40 is disposed between the insulative cover 36 and the metal rod 37, thereby rendering the tube 31 liquid-tight. A connection 41 is attached to the insulative cover 36. The connection 41 communicates with the interior of the tube 31 through passages both in the insulative cover 36 and the connecting member 35. The proximal end of the metal rod 37 is connected to a terminal 42 which is connected to a high frequency electrical power source 44. The outer periphery of the terminal 42 makes screw engagement with a hollow cylindrical cover 43 made of an electrically insulative material such as a synthetic resin. The cover 43 surrounds the terminal 42.

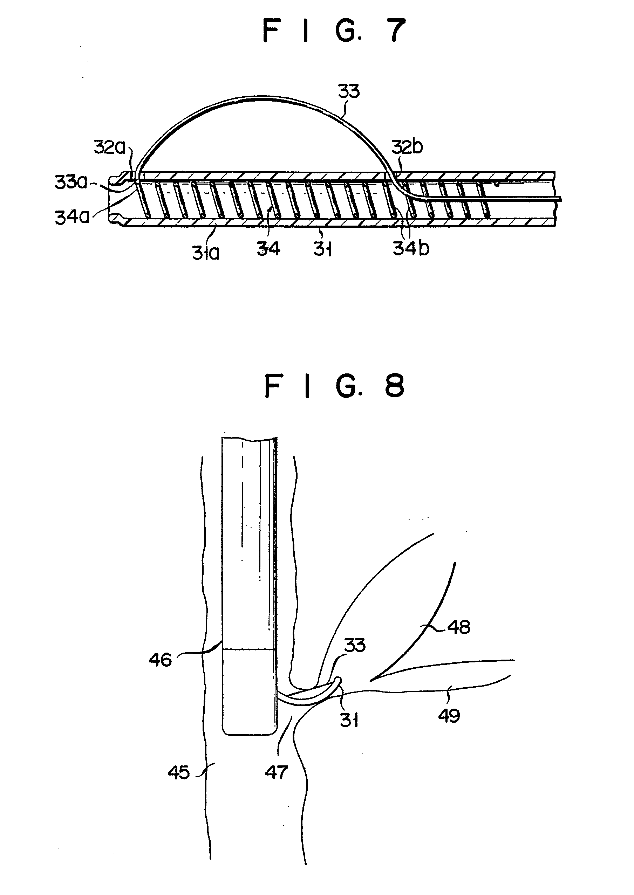

Now it will be described how to operate the above-described first embodiment of the invention. First, the cover 36 is held by, for example, a left hand, and the terminal 42 is connected to the high frequency power source 44. The tube 31 is then inserted into the forceps channel of an endoscope 46 which is, as shown in Fig. 8, inserted in the duodenum 45 in preparation for cutting the duodenal papilla 47. The tube 31 is fed until its distal end portion 31a protrudes from the forceps outlet of the endoscope 46. This done, the metal rod 37 is pulled by operating the cover 43. As the rod 37 is pulled, the electrode wire 33 is drawn, thereby bending the distal end portion 31a of the tube 31 as illustrated in Fig. 6. The distal end portion 31a is bent in the form of a bow, with the wire 33 stretched like a bow string outside the tube 31. The electrode wire 33 is brought into contact with the duodenal papilla 47 as shown in Fig. 8. Then, as in the conventional method, an electrode (not shown) is placed on the patient's skin at the nearest position to the duodenal papilla 47. A high frequency electrical current is supplied to the electrode wire 33 so that the high frequency electrical current flows to the electrode (not shown). Thus, the electrode wire 33 heat-cuts the duodenal papilla 47. Located near the duodenal papilla 47 are the cystic duct 48 and the pancreatic duct 49 as shown in Fig. 8.

Since the reinforcing coil 34 is disposed within the distal end portion 31a of the tube 31, the coil 34 prevents the distal end portion 31a from collapsing or being bent sharply, even if the electrode wire 33 is drawn strongly. This makes the electrosurgical instrument easy to operate and enhances the durability of the instrument.

The wire 33 passes through a gap between the two adjacent turns 34b of the reinforcing coil which are adjacent to the second hole 32b, and its distal end 33a is connected to the distal end 34a of the reinforcing coil 34 adjacent to the first hole 32a. Thus, the tension of the wire 33 is chiefly exerted on the turns 34b of the coil 34 and the distal end of the coil 34. Thus, the holes 32a, 32b are not cut by the heat and the tension of the wire 33 so as to form axially elongated slits.

In order to cut an affected part, the electrode wire 33 may be fed so as to be curved outside the tube 31 as shown in Fig. 7, instead of being pulled to bend the distal end portion 31a of the tube 31. Also in this case, the curved portion of the wire 33 is brought into contact with the affected part.

Through the connector 41, a liquid such as a contrast medium agent may be introduced into the tube 31 and may be sprayed into a coeliac cavity from the distal end of the tube 31.

If the gap between any two adjacent turns 34b of the reinforcing coil 34 is too small, the electrode wire 33 cannot be pulled or fed smoothly. If it is too large, the coil 34 cannot smoothly guide the electrode wire 33 and cannot sufficiently reinforce the tube 31. It is therefore desired that the gap between any two adjacent turns should be about two times the diameter of the wire 33 or a little smaller.

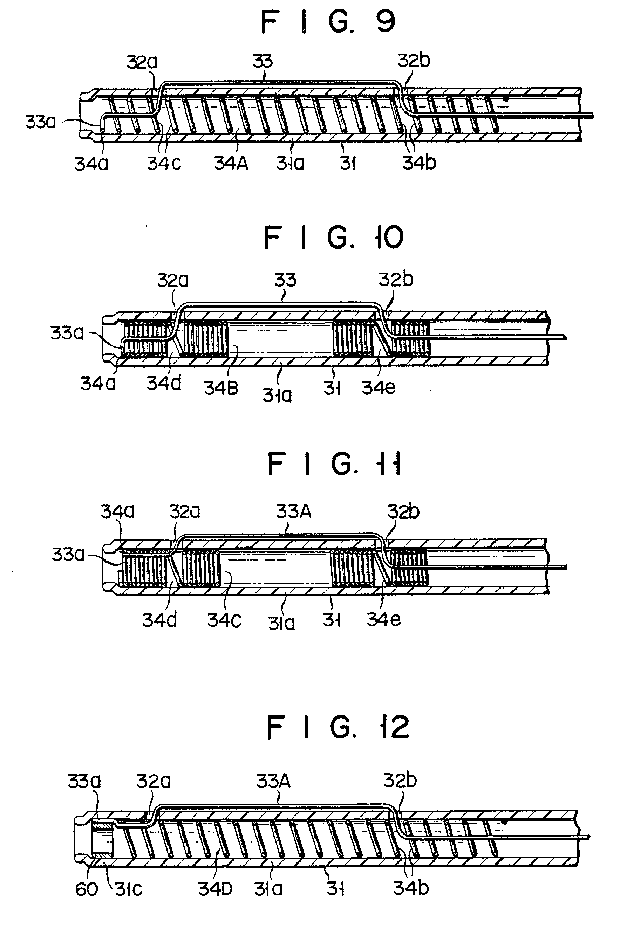

Fig. 9 shows the second embodiment of the invention. In this embodiment a reinforcing coil 34A extends toward the distal end of a tube 31 beyond a first hole 32a of the tube 31. The distal end portion of an electrode wire 33 comes out of the tube 31A through a gap between two adjacent turns 34C of the coil 34A which are adjacent to the first hole 32a and then through the first hole 32a. When the wire 33 is pulled, the wire 33 chiefly contacts these turns 34C. The tension of the wire 33 thus pulled is exerted mainly on the turns 34C. The wire 33 therefore does not cut the tube 31A to form a slit extending from the first hole 32a in the axial direction of the tube 31A.

Fig. 10 shows the third embodiment of this invention. This electrosurgical instrument has a reinforcing coil 34B which portions where turns are is wound densely except that the portions 34d and 34e are sparsely wound. The reinforcing coil 34B is so positioned as to have the sparse portions 34d and 34e located adjacent to electrode wire guiding holes 32a and 32b, respectively. An electrode wire 33 of a tube 31 through the sparse portion 34d and the first hole 32a and then reenters the tube 31 through the second hole 32b and the sparse portion 34e. Since its turns are densely arranged over a greater part of its length, the reinforcing coil 34B is stronger than those used in the first and second embodiments and can more effectively prevent the tube 31 from collapsing.

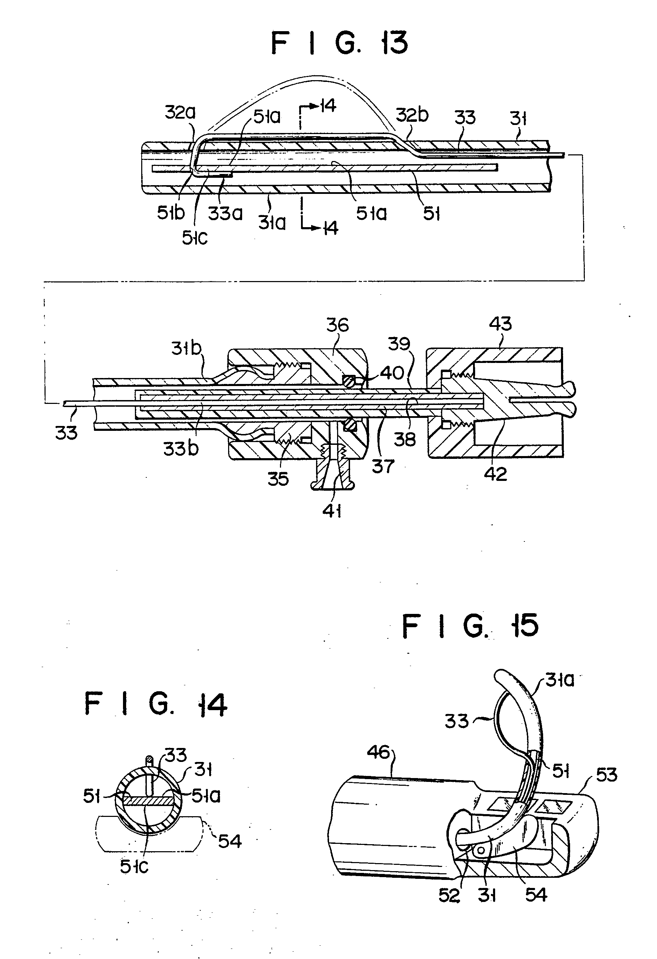

Fig. 11 shows the fourth embodiment of this invention. This electrosurgical instrument is provided with a reinforcing coil 34C which is identical in structure with the coil 34B of the third embodiment. The instrument has an electrode wire 33A which is not integral with the coil 34C and which can thus have a diameter different from that of the metal wire constituting the coil 34C. The distal end 34a of the electrode wire 33A is fixedly connected to the inner periphery of the distal end portion 34a of a reinforcing coil 34C. The fourth embodiment is advantageous in that the reinforcing coil 34C can be made of a suitable material and can hve a suitable size so as to effectively prevent a tube 31 from collapsing, regardless of the material or diameter of the electrode wire 33A.

In the embodiments of Figs. 10 and 11, the gap between the adjacent turns of the sparse portions 34d, 34e of the reinforcing coils 34B, 34C are substantially as twice as the diameter of the wire elements consisting of the coils.

Fig. 12 shows the fifth embodiment of this invention. In this embodiment an electrode wire 33A and a reinforcing coil 34D are separate members. The distal end 33a of the wire 33A is secured to the outer periphery of a ring 40 which is securely provided in the distal end portion 31c of a flexible tube 31. Further the distal end 33a is clamped between the ring 60 and the inner periphery of the tube 31. The wire 33A and the coil 34D are provided independently in order to obtain the same merit as in the fourth embodiment.

Except for the points mentioned above, the second to fifth embodiments shown in Figs. 9 to 12 are identical in construction with the first embodiment shown in Figs. 5, 6 and 7. Like and similar members of the second to fifth embodiments are identified with the same numerals as used to denote the corresponding members of the first embodiment. And the description of these members is omitted.

According to this invention, that portion of an electrode wire which is located outside a tube may be chemically etched to have a smaller diameter than the remaining portion, no matter whether the wire is integral with a reinforcing coil.

The metal wire which constitutes the electrode wire and the reinforcing coil need not to have a circular section. A metal wire having another sectional profile such as a rectangular section may be used instead.

Figs. 13 to 16 show another embodiment which has an elastic plate 51 made of metal such as stainless steel, which is disposed in the distal end portion 31a of a flexible tube 31 and which is used in place of the reinforcing coil used in the embodiment of Fig. 5. The elastic plate 51 extends in the axial direction of the tube 31 and has its ends located beyond a first hole 32a and a second hole 32b of the tube 31. The plate 51 is positioned in the tube 31 so that one broad face 51a of the plate 51 faces the holes 32a and 32b. The plate 51 has a through hole 51b formed in that portion which is adjacent to the first hole 32a. The distal end 33a of an electrode wire 33 passes through the through hole 51b, is folded back and is fixed to the other broad face 51c of the plate 51.

The embodiment of Figs. 13 and 14 are identical with that of Fig. 5 with respect to all the other constructional features. Like and similar members of this embodiment are identified with the same numerals as used to denote the corresponding members of the first embodiment shown in Fig. 5. And the description of these members is omitted.

It will now be described how the embodiment of Figs. 13 and 14 is used. A cover 43 is held by a hand, for example, a right hand and pushed toward the tube 31, thereby pushing a metal rod 37 and the electrode wire 33 further into the flexible tube 31. As a result, the wire 33 slides on the face 51a of the elastic plate 51 toward the distal end of the tube 31, and that portion of the wire 33 which lies outside the flexible tube 31 (that is the exposed portion of the wire 33) is curved in the form of an arc as shown by chain lines in Fig. 13. When the cover 43 is pulled away from the tube 31, the wire 33 is pulled toward the proximal end of the tube 31. Consequently, that portion of the wire 33 which lies outside the tube 31 is pulled in the tube 31 from the second hole 32b so as to be straightened as shown by solid lines in Fig. 13.

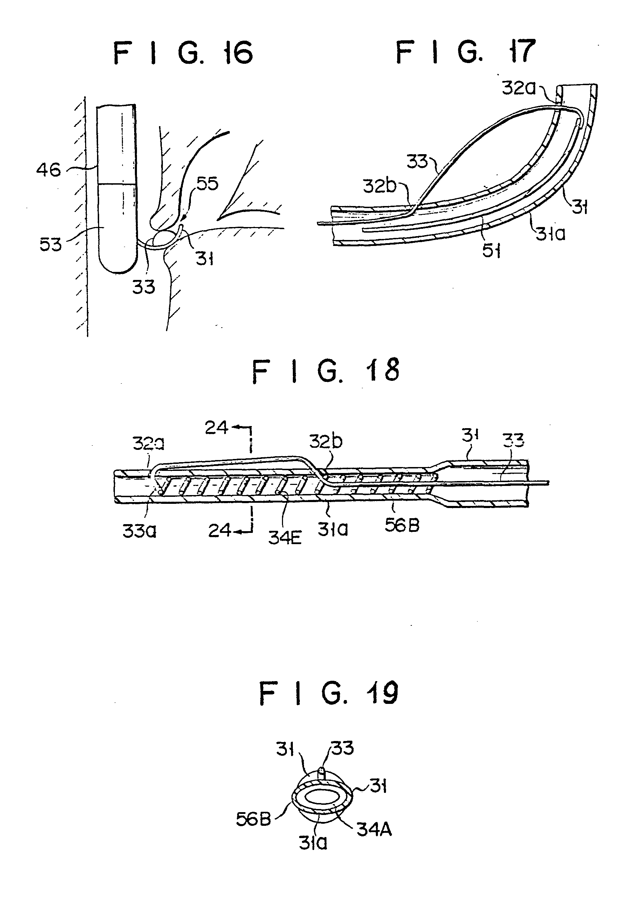

In order to cut, for example, the sphincter of a duodenal papilla, the electrosurgical instrument shown in Figs. 13 and 14 is operated in the following manner. The flexible tube 31 is inserted into a forceps channel of an endoscope 46 until its distal end portion 31a projects from the channel 52 as shown in Figs. 15 and 16, guided by a guide cam 54 which is provided in the distal end portion 53 of the endoscope 46. As long as the distal end portion 31a of the tube 31 projects in this way, the elastic plate 51 remains parallel to the guiding surface of the guide cam 54. An arcuate exposed portion of the wire 33 projects in the direction perpendicular to the broad face 51a of the elastic plate 51. Thus the arcuate portion of the wire 33 projects in the direction perpendicular also to the guiding surface of the guide cam 54. The distal end portion 53 of the endoscope 46 is so moved as to bring the distal end portion 31a of the tube 31 to the sphincter 55 of a duodenal papilla. Once the distal end portion 31a is placed near the sphincter 55, the electrode wire 33 is pushed until there is formed such a high arcuate portion of the wire 33 that touches the sphincter 55. With the arcuate portion of the wire 33 contacting the sphincter 55 as shown in Fig. 16, the terminal 42 is connected to a high frequency source (not shown), thus supplying the wire 33 with a high frequency current. Then the arcuate portion of the wire 33 functions as an electric knife to cut the sphincter 55 of the duodenal papilla. After the sphincter 55 has been cut, the electrode wire 33 is pulled toward the proximal end of the tube 31. Consequently, that portion of wire 33 which lies outside the tube 31 is straightened, and there no longer exists an arcuate portion of the wire 33. Even if the wire 33 is pulled too strongly, the distal end portion 31 of the tube 31 will not be collapsed at the middle point since it is reinforced by the elastic plate 51.

In the embodiment of Figs. 13 and 14, the plate 51 is disposed in the distal end portion 31a of the flexible tube 31 in order to cause an arcuate portion of the wire 33 to project from the tube 31 in a desired direction. The plate 51 may be permanently curved as illustrated in Fig. 17 so as to ensure that the arcuate portion of the wire 33 projects in a desired direction.

The electrode wire 33 need not be fixed in the elastic plate 51. It may be secured in the flexible tube 31.

Further, as shown in Figs. 18 and 19, the whole distal end portion 31a may be made into a guiding portion 56B which has an elliptical sectional profile, and an elastic helical coil 34E having an elliptical sectional profile complementary to the cross section of the interior of the guiding portion 56B may be inserted in the guiding portion 56B. In this case, the distal end 33a of the electrode wire 33 is either connected to the distal end of the coil 34E or is integral with the coil 34E.

It is possible with the embodiment of Fig. 18 to allow an arcuate portion of the wire 33 to project in a desired direction even if a small length of the wire 33 is fed outside the tube 31 through the second hole 32b of the tube 31.

In the embodiment of Figs. 18 and 19 the coil 34A prevents the distal end portion 31a of the tube 31 from being collapsed at the middle point.

Moreover, the entire flexible tube 31 may have an elliptical sectional profile. In this case, too, an arcuate portion of the wire 33 projects in a desired direction even if a small length of the wire 33 is fed outside the tube 31 through the second hole 32b of the tube 31.

## Claims

1. A high frequency electrosurgical instrument for use in an endoscope comprising a flexible tube (31) having a distal end portion (31a) which has first and second wire guiding holes (32a, 32b) set apart axially of the tube (31), the first hole being closer to the distal end than the second hole; an electrode wire (33) extending through said tube (31), one end (33a) of which is fixed within the distal end portion (31a) and the other portion of which comes out of the tube (31) through the first wire guiding hole (32a) to extend outside the tube (31) and enters the tube (31) through the second wire guiding hole (32b) to extend through the tube (31) toward the proximal end of the tube (31), characterized in that an elastic helical coil (34; 34A; 34B; 34C; 34D; 34E) whose cross sectional profile is complementary to that of the interior of said distal end portion (31a) is provided in the distal end portion (31a) so as to prevent the distal end portion (31a) from being permanently deformed and that that portion of said electrode wire (33) which enters the tube (31) passes between first two adjacent turns (34b) of the coil (34; 34A—E), which are separated from each other by a distance larger than the diameter of said electrode wire (33) and then extends through said coil (34; 34A—E) and that said second wire guiding hole (32b) extends between said first two adjacent turns (34b) of the coil (34; 34A—E) such that the length of the second guiding hole (32B) along the tube (31) is larger than the diameter of said electrode wire (33).

2. An instrument according to claim 1, characterized in that said first guiding hole (32a) extends between second adjacent two turns (34c) of said Coil (34; 34A—E) and has a length substantially equal to the gap between said second adjacent two turns (34c) of said coil (34; 34A—E).

3. An instrument according to claim 2, characterized in that the gaps between said first and second adjacent turns (34c) are substantially twice the diameter of said electrode wire (33) and the other portion of said coil (34B; 34C)˙on both outersides of said first and second adjacent two turns (34b) is densely wound.

4. An instrument according to claim 1 or claim 2, characterized in that each adjacent turn of said coil (34; 34A—E) is separated from each other substantially twice the diameter of said electrode wire (33).

5. An instrument according to anyone of claims 1 to 4, characterized in that said electrode wire (33) is integral with said coil (34; 34A—C; 34E).

6. An instrument according to anyone of claims 1 to 5, characterized in that said flexible tube (31) includes a guiding portion (56B) which extends from the proximal end of the distal end portion (31a) toward the proximal end of said tube (31) and which has an elliptical sectional profile with a minor axis extending substantially parallel to the direction in which an portion of said electrode wire (33) extends outside said flexible tube (31).

7. An instrument according to anyone of claims 1 to 4, characterized in that a part or the whole length of said distal end portion (31a) has an elliptical sectional profile with a major axis extending substantially perpendicularly to the direction in which a portion of said electrode wire (33) extends outside said flexible tube (31).

8. A high frequency electrosurgical instrument for use in an endoscope comprising a flexible tube (31) having a distal end portion (31a) which has first and second guiding holes (32a, 32b) set apart axially of the tube (31); the first hole being closer to the distal end than the second hole and an electrode wire (33) extending through said tube (31), one end (33a) of which is fixed within said distal end portion (31a) of the tube (31) and the other portion of which comes out of the tube (31) through the first wire guiding hole (32a) to extend outside the tube (31) and enters the tube (31) through the second wire guiding hole (32b) to extend through the tube (31) toward the proximal end of the tube (31), characterized in that a permanent deformation preventing means is provided in the distal end portion (31a), and that said permanent deformation preventing means is an elastic plate (51) which is disposed in the distal end portion (31a) of the tube (31) between the first and second wire guiding holes (32a, 32b) and which has its faces (51a, 51c) perpendicular to the direction in which the portion of the electrode wire (33) extends outside the tube (31).

9. An instrument according to claim 8, characterized in that the distal end (33a) of said electrode wire (33) is fixed to that portion of said elastic plate (51) which is adjacent to the first wire guiding hole (32a).

10. An instrument according to claim 8, characterized in that the distal end of said elastic plate (51) is located adjacent to the first wire guiding hole (32a) and the distal end (33a) of said electrode wire (33) is fixed to the distal end of said elastic plate (51).

11. An instrument according to anyone of claims 1 to 10, characterized in that a ring member (60) is disposed within the distal end of said tube (31), and the distal end (33a) of said electrode wire (33) is fixed to the ring member (60).

**Patentansprüche**

1. Elektrochirurgisches Hochfrequenzinstrument zur Verwendung in einem Endoskop, bestehend aus einem biegsamen Schlauch (31) mit einem distalen Endstück (31a), welches erste und zweite Drahtführungslöcher (32a, 32b) aufweist, die in Längsrichtung des Schlauchs (31) mit Abstand voneinander angeordnet sind, wobei das erste Loch näher am distalen Ende liegt als das zweite Loch; aus einem durch den Schlauch (31) hindurch laufenden Elektrodendraht (33), dessen eines Ende (33a) innerhalb des distalen Endstücks (31a) befestigt ist, während sein anderes Ende durch das erste Drahtführungsloch (32a) aus dem Schlauch (31) heraustritt, um außen am Schlauch (31) entlangzulaufen und durch das zweite Drahtführungsloch (32b) in den Schlauch (31) einzutreten und sodann durch den Schlauch (31) hindruch zum proximalen Ende des Schlauchs (31) hinzulaufen, dadurch gekennzeichnet, daß im distalen Endstück (31a) eine elastische schraubenförmige Wendel (34; 34A; 34B; 34C; 34D; 34E) mit einem zum Inneren des distalen Endstücks (31a) komplementären Querschnittsprofil vorgesehen ist, um eine bleibende Verformung des distalen Endstücks (31a) zu verhindern, und daß derjenige Teil des Elektrodendrahts (33), welcher in den Schlauch (31) eintritt, zwischen zwei ersten benachbarten Windungen (34b) der Wendel (34; (34A—E) hindurchgeht, die um einen Abstand auseinanderliegen, der größer als der Durchmesser des Elektrodendrahts (33) ist, und dann durch die Wendel (34; 34A—E) hindurchläuft, und daß das zweite Drahtführungsloch (32b) derart zwischen den ersten beiden benachbarten Windungen (34b) der Wendel (34; 34A—E) verläuft, daß die Länge des zweiten Drahtführungslochs (32b) in Längsrichtung des Schlauchs (31) größer als der Durchmesser des Elektrodendrahts (33) ist.

2. Instrument nach Anspruch 1, dadurch gekennzeichnet, daß das erste Führungsloch (32a) zwischen zweiten benachbarten zwei Windungen (34c) der Wendel (34; 34A—E) verläuft und eine Länge hat, die im wesentlichen gleich dem Spalt zwischen den zweiten benachbarten beiden Windungen (34c) der Wendel (34; 34A—E) ist.

3. Instrument nach Anspruch 2, dadurch gekennzeichnet, daß die Spalte zwischen den ersten und zweiten jeweils benachbarten Windungen (34b) im wesentlichen doppelt so groß wie der Durchmesser des Elektrodendrahts (33) sind, und daß der übrige Teil der Wendel (34B; 34C) dicht gewickelt ist.

4. Instrument nach Anspruch 1 oder 2, dadurch gekennzeichnet, daß von einander benachbarten Windungen der Wendel (34; 34A—E) jede Windung von jeder anderen einen Abstand hat, der im wesentlichen gleich dem doppelten Durchmesser des Elektrodendrahts (33) ist.

5. Instrument nach einem der Ansprüche 1 bis 4, dadurch gekennzeichnet, daß der Elektrodendraht (33) und die Wendel (34; 34A—C; 34E) ein Teil bilden.

6. Instrument nach einem der Ansprüche 1 bis 5, dadurch gekennzeichnet, daß der biegsame Schlauch (31) ein Führungsstück (56B) aufweist, welches von proximalen Ende des distalen Endstücks (31a) zum proximalen Ende des Schlauchs (31) hin verläuft und ein elliptisches Querschnittsprofil mit der kleineren Achse im wesentlichen parallel zu derjenigen Richtung aufweist, in welcher ein Teil des Elektrodendrahts (33) außen am biegsamen Schlauch (31) verläuft.

7. Instrument nach einem der Ansprüche 1 bis 4, dadurch gekennzeichnet, daß ein Teil oder die ganze Länge des distalen Endstücks (31a) ein elliptisches Querschnittsprofil mit der größeren Achse im wesentlichen rechtwinklig zu derjenigen Richtung aufweist, in welcher ein Teil des Elektrodendrahts (33) außen am biegsamen Schlauch (31) verläuft.

8. Elektrochirurgisches Hochfrequenzinstrument zur Verwendung in einem Endoskop, bestehend aus einem biegsamen Schlauch (31) mit einem distalen Endstück (31a), welches ersten und zweite Drahtführungslöcher (32a, 32b) aufweist, die in Längsrichtung des Schlauchs (31) mit Abstand voneinander angeordnet sind; wobei das erste Loch näher am distalen Ende liegt als das zweite Loch, und durch den Schlauch (31) hindurch ein Elektrodendraht (33) verläuft, dessen eines Ende (33a) innerhalb des distalen Endstücks (31a) des Schlauchs (31) befestigt ist, während das andere Ende durch das erste Drahtführungsloch (32a) aus dem Schlauch (31) heraustritt, um außen am Schlauch (31) entlangzulaufen und durch das zweite Drahtführungsloch (32b) in den Schlauch (31) einzutreten und sodann durch den Schlauch ·(31) hindurch zum proximalen Ende des Schlauchs (31) hinzulaufen, dadurch gekennzeichnet, daß im distalen Endstück (31a) eine die bleibende Verformung hindernde Einrichtung vorgesehen ist, die aus einer elastischen Platte (51) besteht, welche im distalen Endstück (31a) des Schlauchs (31) zwischen den ersten und zweiten Drahtführungslöchern (32a, 32b) angeordnet ist und deren Flächen (51a, 51c) rechtwinklig zu derjenigen Richtung liegen, in der ein Teil des Elektrodendrahts (33) außen am Schlauch (31) verläuft.

9. Instrument nach Anspruch 8, dadurch gekennzeichnet, daß das distale Ende (33a) des Elektrodendrahts (33) an demjenigen Teil der elastischen Platte (51) befestigt ist, der neben dem ersten Drahtführungsloch (32a) gelegen ist.

10. Instrument nach Anspruch 8, dadurch gekennzeichnet, daß das distale Ende der elastischen Platte (51) neben dem ersten Drahtführungsloch (32a) liegt und das distale Ende (33a) des Elektrodendrahts (33) am distalen Ende der elastischen Platte (51) befestigt ist.

11. Instrument nach einem der Ansprüche 1 bis 10, dadurch gekennzeichnet, daß innerhalb des distalen Endes des Schlauchs (31) ein Ring (60) angeordnet ist, und daß das distale Ende (33a) des Elektrodendrahts (33) am Ring (60) befestigt ist.

## Revendications

1. Instrument électrochirurgical à haute fréquence pour utilisation dans un endoscope comprenant un tube flexible (31) qui présente une partie terminale distale (31a) ayant un premier et un second trou (32a, 32b) de guidage du fil espacés axialement du tube (31), le premier trou étant plus proche de l'extrémité distale que le second trou; un fil électrode (33) s'étendant à travers ledit tube (31), dont une extrémité (33a) est fixée à l'intérieur de la partie terminale distale (31a) et dont l'autre partie sort du tube (31) à travers le premier trou (32a) de guidage du fil pour s'étendre à l'extérieur du tube (31), et pénètre dans le tube (31) à travers le second trou (32b) de guidage du fil pour s'étendre à travers le tube (31) vers l'extrémité proximale du tube (31), caractérisé en ce qu'une bobine hélicoidale élastique (34; 34A; 34B; 34C; 34D; 34E) dont le profil de section transversale est complémentaire de celui de l'intérieur de ladite partie terminale distale (31a) est montée dans la partie terminale distale (31a) de manière à empêcher la déformation permanente de la partie terminale distale (31a) et la partie dudit fil électrode (33) qui pénètre dans le tube (31) passe entre les deux premières spires adjacentes (34b) de la bobine (34; 34A— 34E), qui sont śparées l'une de l'autre d'une distance supérieure au diamètre dudit fil électrode (33) et ensuite s'étend à travers ladite bobine (34; 34A—34E) et en ce que ledit second trou de guidage (32b) du fil passe entre lesdites deux premières spires adjacentes (34b) de la bobine (34; 34A—E) de manière que la longueur du second trou de guidage (32b) le long du tube (31) soit plus grande que le diamètre dudit fil électrode (33).

2. Instrument selond la revendication 1, caractérisé en ce que ledit trou de guidage (32a) s'étend entre deux secondes spires adjacentes (34c) de ladite bobine (34; 34A—E) et a une longueur sensiblement égale à l'intervalle entre lesdite deux secondes spires adjacentes (34c) de ladite bobine (34; 34A—E).

3. Instrument selon la revendication 2, caractérisé en ce que les intervalles entre lesdites premières et secondes spires adjacentes (34c) sont sensiblement égaux à deux fois le diamètre dudit fil électrode (33) et que la partie de ladite bobine (34B, 34C) autre que lesdites première et seconde spires adjacentes (34c) est enroulée de façon dense.

4. Instrument selon la revendication 1 ou 2, caractérisé en ce que chaque spire adjacente de ladite bobine (34; 34A—E) est séparée de chaque autre spire d'une distance que est sensiblement le double du diamètre du fil électrode (33).

5. Instrument selon l'une quelconque des revendications 1 à 4, caractérisé en ce que ledit fil électrode (33) est d'un seul tenant avec ladite bobine (34; 34A—C; 34E).

6. Instrument selon l'une quelconque des revendications 1 à 5, caractérisé en ce que ledit tube flexible (31) comporte une portion de guidage (56B) qui s'étend depuis l'extrémité proximale de la partie terminale distale (31a) verse l'extrémité proximale dudit tube (31) et qui présente un profil de section elliptique dont le petit axe s'étend à peu près parallèllement aus sens dans lequel une portion du fil électrode (33) s'étend à l'extérieur dudit tube flexible (31).

7. Instrument selon l'une quelconque des revendications 1 à 4, caractérisé en ce qu'une partie ou la longueur totale de ladite partie terminale distale (31a) présente un profil de section elliptique dont le grand axe s'étend à peu près perpendiculairement à la direction dans laquelle une partie dudit fil électrode (33) s'étend à l'extérieur dudit tube flexible (31).

8. Instrument électrochirurgical à haute fréquence pour utilisation dans un endoscope comprenant un tube flexible (31) qui présente une partie terminale distale (31a) ayant un premier et un second trou de guidage (32a, 32b) espacés dans le sens axial du tube (31); le premier trou étant plus proche de l'extrémité éloignée que le second trou et un fil électrode (33) s'étendant à travers ledit tube (31), dont une extrémité (33a) est fixée à l'intérieur de la partie terminale distale (31a) du tube (31) et dont l'autre partie sort du tube (31) à travers le premier trou (32a) de guidage du fil pour s'étendre à l'extérieur du tube (31) et pénètre dans le tube (31) à travers le second trou de guidage (32b) pour s'étendre à travers le tube (31) vers l'extrémité proximale du tube (31), caractérisé en ce que des moyens d'empêchement de déformation permanente sont prévus dans la partie terminale distale (31a), et en ce que lesdits moyens d'empêchement de déformation permanente sont une plaque élastique (51) disposée dans la partie terminale distale (31a) du tube (31) entre les premier et second trous (32a, 32b) de guidage du fil et dont les faces (51a, 51c), sont perpendiculaires à la direction dans laquelle la partie du fil électrode (33) s'étend à 'extérieur du tube (31).

9. Instrument selon la revendication 8, caractérisé en ce que l'extrémité distale (33a) du fil électrode (33) est fixée à la partie de la plaque élastique (51) qui est adjacente au premier trou (32a) de guidage du fil.

10. Instrument selon la revendication 8, caractérisé en ce que l'extrémité distale de ladite plaque élastique (51) est disposée de façon adjacente au premier trou (32a) de guidage du fil et l'extrémité distale (33a) du fil électrode (33) est fixée à l'extrémité distale de ladite plaque élastique (51).

11. Instrument selon l'une quelconque des revendications 1 à 10, caractérisé en ce qu'un organe annulaire (60) est disposé dans l'extrémité distale dudit tube (31) et l'extrémité éloignée (33a) du fil électrode (33) est fixée à l'organe annulaire (60).

0 019 930

# F I G. 1

# F I G. 2

# F I G. 3

# F I G. 4

# F I G. 5

# F I G. 6

# F I G. 7

# F I G. 8

0019930

# F I G. 9

# F I G. 10

# F I G. 11

# F I G. 12

# F I G. 13

32a  51a  →14  32b  33  31

51b  33a  31a  ↓14  51a  51

51c

36  39  43

31b  40

33  38

33b  37  42

35  41

# F I G. 15

31a

33  51  53

46  52  31  54

# F I G. 14

33 31

51  51a

54

51c

# F I G. 16

# F I G. 17

# F I G. 18

# F I G. 19